# EUROPÄISCHE PATENTSCHRIFT

(11) **EP 4 563 103 B1**
(45) Veröffentlichungstag und Bekanntmachung des Hinweises auf die Patenterteilung: **19.08.2026**
(21) Anmeldenummer: 24215791.5
(22) Anmeldetag: 27.11.2024
(51) Int. Cl.: A61B 17/70

(54) **ZENTRIEREINHEIT MIT VERRIEGELUNGSSCHRAUBE**
CENTRING UNIT WITH LOCKING SCREW
ENSEMBLE DE CENTRAGE AVEC VIS DE VERROUILLAGE

(30) Priorität: 28.11.2023 DE 102023133254
(43) Veröffentlichungstag der Anmeldung: 04.06.2025
(73) Patentinhaber: Aesculap AG, 78532 Tuttlingen (DE)
(72) Erfinder: LINKE, Ralph, 78234 Engen (DE); RICCI, Denis, 78573 Wurmlingen (DE)
(74) Vertreter: Winter, Brandl - Partnerschaft mbB

(56) Entgegenhaltungen:
- WO-A1-2022/184798
- WO-A2-2008/024937
- US-A1- 2011 184 469
- US-A1- 2017 143 385
- US-A1- 2022 133 360

## Beschreibung

Die vorliegende Offenbarung betrifft eine Zentriereinheit für ein Montagewerkzeug vorzugsweise in Form eines Schraubendrehers, welches für das Setzen einer Madenschraube in die Tulpe einer Pedikelschraube vorgesehen und ausgebildet ist.

### Hintergrund der Offenbarung

Grundsätzlich existieren im Stand der Technik mono- sowie polyaxiale Pedikelschrauben, die zum Eindrehen in einen Wirbelknochen vorgesehen sind. Hierfür weißt eine solche Pedikelschraube grundsätzlich einen mit einem Außengewinde versehenen Schaft auf, an dessen proximalem Ende/Endabschnitt eine sogenannte Tulpe fixiert (monoaxiale Ausgestaltung) oder beweglich, nämlich schwenk- und drehbar an einem distalen Schaftkopf gelagert ist (polyaxiale Ausgestaltung). Eine solche Tulpe ist, einfacher ausgedrückt, ein Rohrstutzen, dessen Wände an zwei diametral sich gegenüberliegenden Umfangsabschnitten längsgeschlitzt ist, derart, dass sich die Schlitze in Richtung proximal (weg vom Schaft) an der Tulpenstirnseite öffnen und somit jeweils eine U-Form erhalten.

Auf der Tulpeninnenseite weißt der Rohrstutzen ein Innengewinde auf, in das eine sogenannte Madenschraube von der proximalen Stirnseite der Tulpe aus eingedreht oder eindrehbar ist. Die Madenschraube ist/kann gegen einen Verbindungsstab angelegt (werden), welcher in die sich gegenüberliegenden Schlitze eingesetzt ist und wenigstens zwei Pedikelschrauben von zwei in Wirbelsäulen-Längsrichtung beabstandeten Wirbels zu verbinden, um so diese zumindest zwei Wirbel relativ zueinander lagezufixieren. Hierfür drückt die Madenschraube den eingelegten Verbindungsstab gegen das distale Ende der Tulpe (monoaxiale Ausführung) oder gegen den proximalen Schaftkopf (polyaxiale Ausgestaltung) und klemmt diesen fest. Das Innengewinde der Tulpe ist schließlich so gestaltet, dass dieses eine Art Selbsthemmung bewirkt, und somit ein eigenständiges (unbeabsichtigtes) Lösen des festgeklemmten Stabs unwahrscheinlich wird.

Aus Gründen der besseren Handhabbarkeit einer solchen Pedikelschraube während deren Implantierung wird diese zuerst an einem betreffenden Wirbel gesetzt, wofür beispielsweise ein Inbusschlüssel durch die, an der proximaler Stirnseite offenen Tulpe hindurchgeführt und in ein entsprechendes Mehrkant-Sackloch am proximalen Endabschnitt des Pedikelschrauben-Schafts eingeführt wird, um den Schaft in den Wirbelknochen einzudrehen. Daraufhin wird der vorstehend genannte Verbindungsstab in die Tulpe eingeführt und die Madenschraube in das Innengewinde der Tulpe eingedreht.

### Stand der Technik

Die DE 10 2011 053 295 A1 offenbart bereits eine Montagehilfe zur Vereinfachung des vorstehend genannten Implantationsprozesses insbesondere bei Verwendung von polyaxialen Pedikelschrauben. Diese Montagehilfe hat im Prinzip eine Innenhülse, welche innseitig der Tulpe axial gegen den proximalen Schaftkopf angedrückt wird. Des Weiteren hat die Montagehilfe eine Außenhülse, welche die Tulpe außenseitig umgibt und in eine außenseitige Tulpenkulisse oder Nut eingreift. Schließlich ist ein Verspannmittel beispielsweise in Form einer Überwurfmutter vorgesehen, über welche die Innenhülse relativ zur Außenhülse axial verstellbar ist. Damit ist es möglich die zunächst mit Bezug zum Schaft bewegliche Tulpe temporär am proximalen Schaftkopf zu verspannen und so eine vorläufig gewählte Tulpenausrichtung einzufrieren. Danach kann der Verbindungstab in die U-förmigen Schlitze eingeführt und die Madenschraube eingedreht werden. Erst nach Abschluss dieses Montagevorgangs wird das Verspannmittel wieder gelöst und die gesamte Montagehilfe von der Tulpe abgenommen. Damit ist der Implantationsprozess beendet.

Grundsätzlich kann die vorstehend genannte Montagehilfe auch dazu beitragen, das Einführen/Aufsetzen der Madenschraube in/auf das Innengewinde der Tulpe zu erleichtern, indem sie ein Verkanten der Madenschraube verhindert, zumindest aber erschwert. Jedoch muss das Werkzeug zum Festdrehen der Madenschraube in die bereits verspannte Innenhülse axial eingeführt werden. Dafür ist es dann aber auch erforderlich, die Madenschraube am Werkzeug provisorisch zu halten und zwar in einer Weise, dass die Madenschraube bei Einführen des Werkzeugs in die Innenhülse nicht versehentlich abfällt oder abgeschlagen werden kann. Dies bereitet im Stand der Technik Probleme.

Eine Zentriereinheit mit den Merkmalen des Oberbegriffs des Anspruchs 1 ist aus der US 2011/184469 A1 bekannt.

### Kurzbeschreibung der Erfindung

Es ist die Aufgabe der vorliegenden Erfindung, eine Montagehilfe in Form oder in Funktion einer Zentriereinheit bereitzustellen, die ein sicheres Aufsetzen/Eindrehen einer Madenschraube auf/in das Innengewinde einer Tulpe vereinfacht.

Diese Aufgabe wird durch eine Zentriereinheit mit den Merkmalen des Anspruchs 1 gelöst. Vorteilhafte Weiterbildungen der Erfindung sind Gegenstand der abhängigen Ansprüche.

### Kurzbeschreibung der Offenbarung

Der Kern der vorliegenden Offenbarung besteht in der Anordnung einer (medizinischen) Zentriereinheit mit einer Außenhülse, in der eine axial wirkende Federeinrichtung (Vorspannmittel) beispielsweise in Form einer Schraubenfeder, einer Anzahl von (axial aufgereihten) Tellerfedern, einer Hülse aus einem elastischen Material (Gummi, Kunststoff etc.) oder dergleichen Vorspannmittel eingelegt/eingesetzt ist, die sich an einem Hülsen-innseitigen Absatz (distaler Federsitz) in Richtung distal abstützt. Die Federeinrichtung ist dafür vorgesehen und ausgebildet, eine axiale Vorspannkraft auf ein in die Außenhülse eingeführtes Werkzeug (das zum Aufsetzen und Festdrehen einer Madenschraube auf/in einer Tulpe einer Pedikelschraube ausgebildet ist) auszuüben, sobald das Werkzeug in der Außenhülse über ein vorbestimmtes Maß (relativ zur Außenhülse) axial verschoben wird. Die offenbarungsgemäße Außenhülse hat einen distalen Anlageabschnitt (distale Stirnkante), der dafür vorgesehen und ausgebildet ist, gegen die Tulpe (als Kraftwiderlager) axial angesetzt zu werden, derart, dass bei einer axialen Kraftbeaufschlagung des Werkzeugs in Richtung distal das Werkzeug gegen die Federvorspannung relativ zur Außenhülse verschiebbar ist, soweit, dass die distale Werkzeugspitze über die distale Stirnkante der Außenhülse (und damit in die Tulpe) vorragen kann (bzw. vorragt).

Im praktischen Einsatz der wie vorstehend gekennzeichneten Zentriereinheit wird diese zunächst auf einen Werkzeugschaft aufgesetzt. Dieser ist vorzugsweise mit einem Axialanschlag (proximaler Federsitz) versehen oder versehbar, dessen Schaftlängsposition so gewählt oder wählbar ist, dass die Außenhülse in Konstruktionslage zum Werkzeugschaft (ohne externe/manuelle axiale Kraftbeaufschlagung des Werkzeugs) die distale Spitze des Werkzeugschafts radialseitig umgibt und bevorzugt sogar axial über die distale Spitze des Werkzeugschafts vorragt. In diesem Zustand kann nun die Madenschraube auf die distale Spitze des Werkzeugschafts aufgesetzt werden, wobei sie durch die (die Werkzeugschaftspitze umfangsseitig umgebende) Außenhülse vor seitlichen/radialen Schlägen oder Stößen geschützt wird. In diesen (vormontierten/vorbereiteten) Zustand wird das Werkzeug samt Madenschraube und diese radial umgebende Außenhülse der Zentriereinheit an die proximale Tulpen-Stirnseite der bereits implantierten Pedikelschraube herangeführt und die Außenhülse axial gegen die Tulpe angedrückt. Dabei wird das Werkzeug gegen die Vorspannkraft des in die Außenhülse eingelegten/eingesetzten Vorspannmittels relativ zur Außenhülse axial verschoben (das Vorspannmittel wird dabei zwischen dem distalen Federsitz an der Außenhülse und dem proximalen Federsitz vorzugsweise am Werkzeugschaft eingespannt), wodurch die Madenschraube über die distale Stirnkante der Außenhülse hinaus (im Wesentlichen verkantungsfrei) in die Tulpe eingeführt wird. Abschließend braucht dann nur noch das Werkzeug innerhalb der Außenhülse (unter Aufrechterhaltung der axialen Andrückkraft) gedreht zu werden, um die Madenschraube innerhalb der Tulpe festzuziehen.

Gemäß einer bevorzugten Ausgestaltung ist am proximalen Endabschnitt der Außenhülse eine Innenhülse axialverschiebbar eingesetzt oder einsetzbar, die sich in distaler Richtung gegen das Vorspannmittel abstützt. Dabei kann sich die Innenhülse proximal an das Vorspannmittel anschließen oder zumindest abschnittsweise in das Vorspannmittel vorzugsweise in Form der Schraubendruckfeder radial innseitig hineinragen, um so bevorzugt das Vorspannmittel radial zu stützen. Im letzteren Fall ist die Innenhülse mit wenigstens einem radial nach außen abstehenden Umfangsvorsprung ausgebildet, der als proximaler Axialanschlag (proximaler Federsitz) für das Vorspannmittel dient. In diesem Fall dient der Axialanschlag am Werkzeugschaft nicht mehr unmittelbar als proximaler Federsitz sondern als Anschlag für die Innenhülse, welche ihrerseits wiederum den proximalen Federsitz ausbildet. Weiter vorzugsweise kann die Innenhülse mit einem zusätzlichen radial nach außen vorragenden proximalen Vorsprung ausgebildet sein, der als Bewegungsanschlag in Richtung proximal dient und mit einer Hinterschneidung an der radialen Innenseite der Außenhülse in Anlage bringbar ist.

Bei dieser bevorzugten Ausgestaltung bildet die Zentriereinheit quasi ein in sich geschlossenes Bauteil, bei welchem die Federeinrichtung (Vorspannmittel) zwischen dem distalen und proximalen Federsitz innerhalb der Zentriereinheit mit oder ohne Vorspannung eingesetzt ist und ein proximales Herausfallen der Federeinrichtung (Vorspannmittel) aus der Außenhülse durch die Innenhülse vermeidbar ist und wobei der Innendurchmesser der Innenhülse auf den Werkzeugschaft derart abgestimmt werden kann, dass dieser mit wenig Spiel in der Innenhülse gelagert ist. Dies trägt zu einer noch sichereren/verbesserten (verkantungsfreien) Eindrehbarkeit der Madenschraube bei.

Gemäß einem bevorzugten, ggf. unabhängig beanspruchbaren Aspekt der Offenbarung sind an der Außenhülse an wenigstens zwei diametral sich gegenüberliegenden Umfangsabschnitten jeweils ein Eingriffsarm schwenkbar gelagert. Jeder Eingriffsarm erstreckt sich dabei in Außenhülsen-Längsrichtung und ist in seinem longitudinalen Mittenabschnitt wippenartig an einem jeweiligen Lagerbock anscharniert, der an der radialen Außenseite der Außenhülse fixiert oder ausgebildet ist. Jeder Eingriffsarm ist mittels einer Feder so vorgespannt, dass dessen distales Ende radial nach innen (und folglich dessen proximales Ende radial nach außen) gedrückt wird. Die Feder kann entweder eine Schraubendruckfeder sein, welche sich proximal zum Lagerbock unterhalf des jeweiligen Eingriffsarms gegen die Außenhülse radial nach außen abstützt oder eine um die Scharnierachse gewundene Spiralfeder sein, deren eine Federende gegen die Außenhülse und deren anderes Federende gegen den jeweiligen Eingriffsarm proximal zum Lagerbock anliegt.

Vorzugsweise hat jeder Eingriffsarm einen distalen Eingriffsabschnitt, der dafür vorgesehen und ausgebildet ist, mit der äußeren Umfangsseite der Tulpe form- und/oder reibschlüssig in Eingriff zu kommen, um die Außenhülse an der proximalen Stirnseite der Tulpe zu halten und ggf. zu zentrieren. Weiter vorzugsweise bildet der zum jeweiligen Lagerbock proximale Armabschnitt eine Betätigungsbranche, wodurch der distale Eingriffsabschnitt gegen die Federvorspannkraft radial nach Außen bewegt werden kann.

Gemäß einer bevorzugten Ausführungsform der Offenbarung bildet die Innenseite der Außenhülse in einem distalen Hülsenabschnitt einen radial aufgeweiteten Aufnahmeraum zur (berührungslosen) Aufnahme der Madenhülse (d.h. der Werkzeugschaftspitze mit aufgesetzter Madenschraube). Weiter vorzugsweise verengt/verjüngt sich der Aufnahmeraum in Richtung proximal zu einem Innenradius, der entweder an den Außenradius des Werkzeugschafts oder der Innenhülse angepasst ist. Ein sich daraus ergebender Außenhülsen-Axialabschnitt mit verengtem Innenradius bildet an seinem proximalen Ende (etwa im axialen Mittenabschnitt der Außenhülse) eine radiale Aufweitung an der Außenhülsen-Innenseite, wodurch sich eine nach proximal gerichtete Anlagekante (distaler Federsitz) für die Federeinrichtung/Vorspannmittel (Schraubenfeder) ergibt. D.h., durch die radiale Aufweitung am proximalen Ende des Außenhülsen-Axialabschnitts mit verengtem Innenradius bildet sich je nach Ausführungsform zwischen der Außenhülse und dem Werkzeugschaft oder zwischen der Außenhülse und der Innenhülse ein Umfangsspalt, in welchen die Federeinrichtung/Vorspannmittel (Schraubenfeder) eingesetzt/eingelegt ist.

Vorzugsweise ist der proximale Federanschlag/Federsitz entweder am Werkzeugschaft oder an der Innenhülse so dimensioniert, dass er umfangsseitig gleitend an der Innenseite der Außenhülse anliegt. Insbesondere für den Fall, dass der proximale Federanschlag/Federsitz ringförmig ist, wird somit die Außenhülse an zwei axial-beabstandeten Stellen, nämlich im Bereich des distalen Außenhülsen-Axialabschnitts mit verengtem Innenradius sowie im Bereich des proximalen Federanschlags/Federsitzes geführt und damit mit Bezug zum eingeführten Werkzeugschaft zentriert.

Gemäß einem weiteren bevorzugten Ausführungsbeispiel der Offenbarung ist am proximalen Endabschnitt der Außenhülse eine Halterung beispielsweise in Form einer Haltehülse befestigbar (beispielsweise durch Verrasten, Verschrauben oder dergleichen lösbare Verbindungen) die dafür vorgesehen ist, quasi Abziehsicherung mit dem Werkzeugschaft in axialen Wirkeingriff gebracht zu werden. Beispielsweise kann die Hülsenmutter radial nach innen vorstehende Klemmfüße oder aufweisen, die ggf. federelastisch über den proximalen Axialanschlag (ggf. Federsitz) am Werkzeugschaft elastisch überstülpbar sind oder die Haltehülse hat einen radial nach innen vorragenden Axialanlageabschnitt, der mit dem proximalen Axialanschlag (ggf. Federsitz) am Werkzeugschaft an dessen proximaler Seite in Anlage bringbar ist, um ein unbeabsichtigtes Abfallen der Zentriereinheit vom bereit eingeführten Werkzeugschaft zu verhindern. Im letztgenannten Fall ist es klar, dass die Haltehülse von der proximalen Seite des proximalen Axialanschlags (ggf. Federsitz) am Werkzeugschaft her montiert werden muss oder zuerst auf den Werkzeugschaft aufgesetzt werden muss bevor der proximale Axialanschlag (ggf. Federsitz) am Werkzeugschaft montiert/fixiert wird.

Weiter vorzugsweise kann die Innenhülse insbesondere an ihrer proximalen Stirnkante mit einem radial nach Außen vorragenden Kragen versehen sein, der die Axialverschiebung der Innenhülse mit Bezug zur Außenhülse entgegen der Vorspannkraft der Federeinrichtung/Federmittels nach distal begrenzt, wenn der Kragen an der proximalen Stirnkante der Außenhülse in Anlage kommt. In diesem Fall kann ein vollständiges Komprimieren der Federeinrichtung vermieden werden.

Weitere vorteilhafte Ausgestaltungen sind Gegenstand der Unteransprüche.

### Kurzbeschreibung der Figuren

Die Offenbarung wird nachfolgend anhand bevorzugter Ausführungsbeispiele unter Bezugnahme auf die begleitenden Figuren näher erläutert.
Fig. 1 zeigt die Perspektivenansicht einer Zentriereinheit gemäß einem ersten bevorzugten Ausführungsbeispiel der vorliegenden Offenbarung,
Fig. 2 zeigt eine Seitenansicht der Zentriereinheit gemäß Fig. 1,
Fig. 3 zeigt einen Längsschnitt der Zentriereinheit gemäß Fig. 1 mit bereits eingeführtem Werkzeugschaft und darauf aufgesetzter Madenschraube sowie mit einer Pedikelschraube im Eingriffszustand mit der Zentriereinheit und dem Werkzeug und
Fig. 4 zeigt die Perspektivenansicht einer Zentriereinheit gemäß einem zweiten bevorzugten Ausführungsbeispiel der vorliegenden Offenbarung.

### Figurenbeschreibung

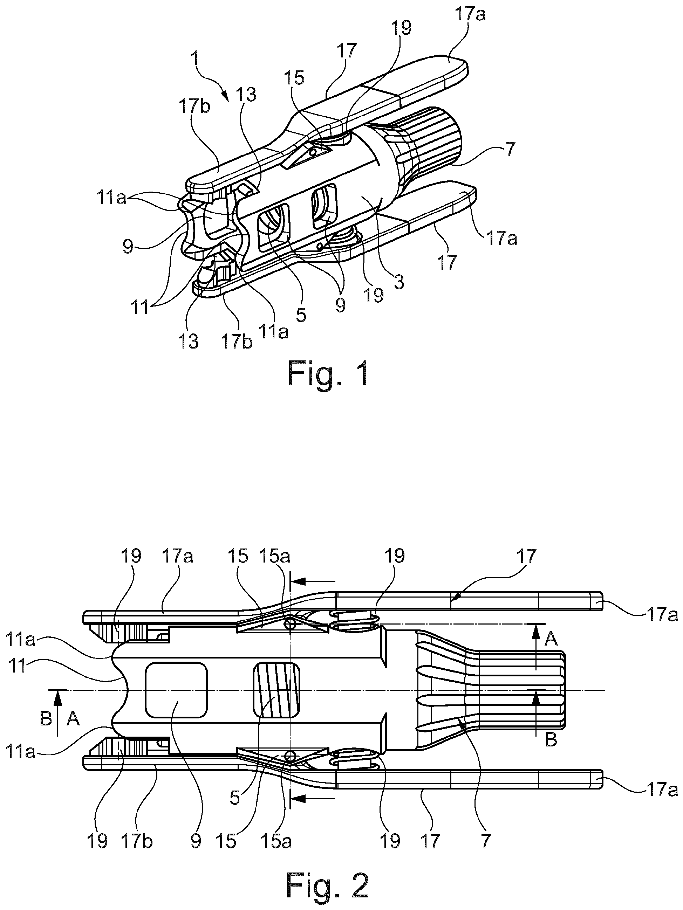

Gemäß dem in Fig. 1 gezeigten ersten bevorzugtes Ausführungsbeispiel der vorliegenden Offenbarung hat die Zentriereinheit 1 eine Außenhülse 3 sowie eine Federeinrichtung (Vorspannmittel) 5, welche in der Außenhülse 3 gelagert ist. Die Außenhülse 3 hat an ihrem proximalen (einer Pedikelschraube abgewandten) Endabschnitt ein außenumfangsseitiges Vielnutprofil 7 vergleichbar zu einem Werkzeugbit, um in die Schaftspitze eines Werkzeugs (Schraubendreher) drehfest eingesetzt zu werden. Ein distaler (einer Pedikelschraube zugewandter) Endabschnitt der Außenhülse 3 weist eine Gitterstruktur auf mit einer Anzahl von radialen Durchbrüchen 9, wodurch ein umfangsseitiger Einblick in das distale Innere der Außenhülse 3 genommen werden kann. Des Weiteren sind an der distalen Stirnkante der Außenhülse 3 zwei diametral sich gegenüberliegende Teilkreisbögen 11 ausgeformt, wodurch sich vier umfangsbeabstandete Abstützecken 11a ausbilden, die sich in Richtung distal erstrecken. In Umfangsrichtung gesehen zwischen den Teilkreisbögen 11 sind an der distalen Stirnkante der Außenhülse 3 zwei folglich ebenfalls diametral sich gegenüberliegende Einkerbungen 13 eingearbeitet, die im Wesentlichen eine rechteckige Form haben und sich bevorzugt weiter in Richtung proximal erstrecken (Kerbtiefe) als die Teilkreisbögen 11.

Im Umfangsbereich der Einkerbungen 13 jedoch in proximalem Abstand zu diesen ist jeweils ein Lagerbock 15 an der äußeren Umfangsseite der Außenhülse 3 angeordnet/ausgebildet. An diesen zwei Lagerböcken 15 ist jeweils ein Eingriffsarm 17 wippenartig anscharniert, die sich co-axial zur Außenhülse 3 erstrecken und durch jeweils eine Feder (Schraubendruckfeder) 19 gemäß der Fig. 1 gegen den Uhrzeigersinn vorgespannt sind. Im Konkreten ist an jedem Eingriffsarm 17 an dessen zum Lagerbock 15 proximalen Armabschnitt 17a die Feder 19 zwischen dem proximalen Armabschnitt 17a und der Außenhülse 3 platziert, welche folglich den proximalen Armabschnitt 17a radial von der Außenhülse 3 wegdrückt, weshalb ein bezüglich des Lagerbocks 15 distaler Armabschnitt 17b radial zu der Außenhülse 3 gedrückt wird.

In der Fig. 2 ist der äußere Aufbau der offenbarungsgemäßen Zentriereinheit 1 noch besser zu erkennen.

Demzufolge sind am distalen Endabschnitt jedes Eingriffsarms 17 bzw. des zugehörigen distalen Armabschnitts 17b sogenannte Eingriffsprofile 19 angeordnet, die dafür vorgesehen und ausgebildet sind, mit der Außenseite einer pedikelschraubenseitigen Tulpe (gemäß vorstehender Definition) in Reib- und/oder Formschluss zu kommen. Dafür sind die Eingriffsprofile 19 so dimensioniert und platziert, dass sie in Konstruktionslage gemäß der Fig. 2 zumindest teilweise in die Einkerbungen 13 radial hineinragen. Dies ist insbesondere in der Fig. 2 gezeigt. Des Weiteren bilden die Eingriffsarme 17 im Längsschnitt quer zur Scharnierachse 15a der Lagerböcke 15 eine Stufe, wodurch der proximale Armabschnitt 17a in Konstruktionslage gemäß der Fig. 2 einen größeren Radialabstand zur Außenhülse 3 erhält als der distale Armabschnitt 17b. Dadurch ist es möglich, bei manuellem Zusammendrücken der beiden gegenüberliegenden proximalen Armabschnitte 17a (stellen in diesem Fall Betätigungsbranchen dar) die beiden gegenüberliegenden distalen Armabschnitte 17b (stellen in diesem Fall Eingriffsabschnitte dar) in Radialrichtung um einen ausreichenden Betätigungsweg auseinanderzubewegen.

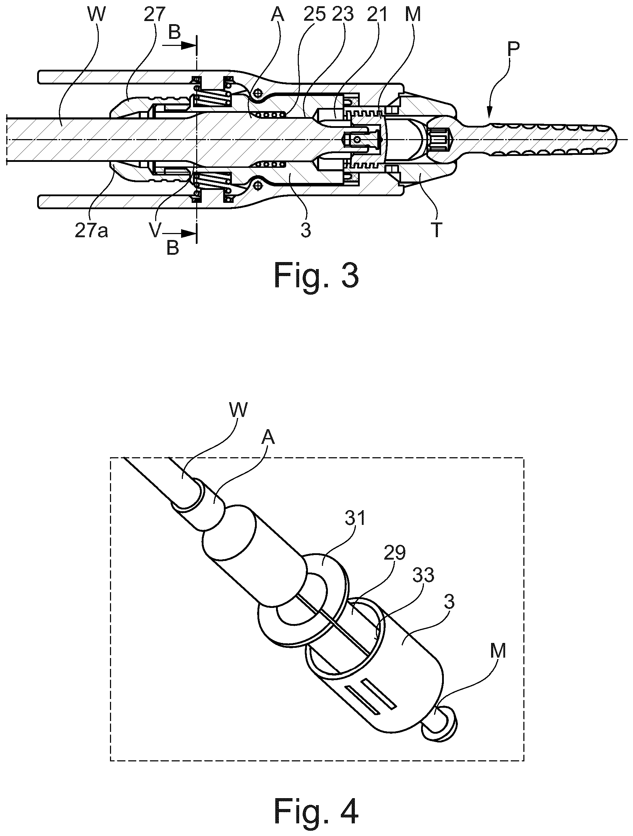

In der Fig. 3 ist der innere Aufbau der offenbarungsgemäßen Zentriereinheit 1 zu erkennen.

Demzufolge bildet die Außenhülse 3 in ihrem distalen Endabschnitt einen nach distal offenen Aufnahmeraum 21 mit erweitertem Durchmesser, der zur zumindest teilweisen, vorzugsweise vollständigen Aufnahme einer Madenhülse M ausgelegt ist, die zum Einschrauben in die Tulpe T einer Pedikelschraube P gemäß dem Stand der Technik vorgesehen ist. Der proximale Endbereich des Aufnahmeraums 21 ist durch einen radial verjüngten Außenhülsen-Axialabschnitt 23 definiert, dessen Innenseite ein Führungsgleitlager für einen eingeführten Werkzeugschaft W darstellt. Der Außenhülsen-Axialabschnitt 23 mit verengtem Innenradius endet proximal unter Ausbildung einer radial nach außen sich erweiternden Anschlagskante 25, die einen distalen Federsitz für die Federeinrichtung 5 vorzugsweise in Form einer Schraubendruckfeder bildet. Die Schraubendruckfeder 5 ist dafür vorgesehen und ausgebildet, mit einer einen proximalen Federsitz definierenden Anschlagskante A auf Seiten des Werkzeugschafts W dann in Anlage zu kommen, wenn der Werkzeugschaft W in Richtung distal in die Außenhülse 3 eingeführt wird. Schließlich ist am proximalen Endabschnitt der Außenhülse 3 ein Rückhaltebauteil 27 montiert beispielsweise durch Aufschrauben oder verrasten an der Außenhülse 3 welche einen radial nach innen vorragenden Halte- oder Anschlagsabschnitt 27a hat. Dieser Halte- oder Anschlagabschnitt 27a ist dafür vorgesehen und dimensioniert, mit einem radial nach außen gerichteten Vorsprung V auf Seiten des Werkzeugschafts W in Richtung distal in Anlage zu kommen, um ein unbeabsichtigtes, ggf. durch die Federvorrichtung 5 ausgelöstes Abfallen der Zentriereinheit 1 vom Werkzeugschaft W zu verhindern.

Die Funktion der offenbarungsgemäßen Zentriereinheit 1 lässt sich wie folgt zusammenfassen:
Zunächst wird das Rückhaltebauteil (Halterung) 27 von proximal her auf den Werkzeugschaft W aufgeschoben, worauf von distal her die Außenhülse 3 auf den Werkzeugschaft W aufgeschoben wird. Das Rückhaltebauteil 27 und die Außenhülse 3 werden miteinander verbunden, wie dies in der Fig. 3 dargestellt ist. In dieser Konstruktionslage ist die Federeinrichtung 5 entspannt oder nur geringfügig vorgespannt, wobei die distale Spitze des Werkzeugschafts W in den Aufnahmeraum 21 ragt. Jetzt kann eine Madenschraube M auf die distale Spitze drehfest aufgesteckt werden.

Das so vorbereitete Werkzeug wird in den OP-Bereich im Patienten geführt und auf die Tulpe T der bereits in den Konchen eingeschraubten Pedikelschraube P axial aufgesetzt. Beispielsweise kommen dabei die Abstützecken 11a mit einer Umfangskante an der Tulpe T in Axialanlage. Gleichzeitig mit dem vorstehend beschriebenen Aufsetzvorgang werden die beiden Eingriffsarme 17 manuell betätigt, sodass die distalen Eingriffsabschnitte /Eingriffsprofile 19 die Tulpe außenumfangsseitig umgreifen, worauf die Eingriffsarme 17 losgelassen werden und durch die Vorspannkraft der Federn 19 gegen die Tulpe T umfangsseitig angedrückt werden. Dabei kommen die Eingriffsprofile 19 mit der Tulpe T in Reib- und/oder Rasteingriff und halten so die Zentriereinheit 1 an der Tulpe T fest.

Schließlich kann das Werkzeug axial zur Tulpe T vorgedrückt werden, wobei die die Federeinrichtung 5 elastisch komprimiert wird. Diese Vordrückbewegung wird durch die Außenhülse 3 geführt, sodass ein Verkanten der Madenschraube M zu Beginn des Eindrehvorgangs nahezu ausgeschlossen ist.

Die Fig. 4 zeigt den äußeren Aufbau einer Zentriereinheit 1 nach einem zweiten bevorzugten Ausführungsbeispiel der Offenbarung.

In diesem Fall ist eine Innenhülse 29 vorgesehen, welche in die Außenhülse 3 von proximal her eingeführt ist. Die Innenhülse 29 hat einen radial nach außen vorragenden Anschlagsvorsprung (nicht weiter gezeigt) vergleichbar zur proximalen Anschlagskante A auf Seiten des Werkzeugschafts W, wie dieser in Fig. 3 dargestellt ist. D.h. in diesem Fall bildet die Innenhülse 29 den proximalen Federsitz für die Federeinrichtung 5 aus, wobei sich die Innenhülse 29 dann an der proximalen Anschlagskante A auf Seiten des Werkzeugschafts W axial abstützen kann.

Die Innenhülse 29 hat in einem proximalen Abschnitt einen umlaufenden Kragen 31, der als Vordrück-Wegbegrenzung dienend mit der proximalen Stirnseite der Außenhülse 3 in Anlage bringbar ist. Schließlich hat die Innenhülse 29 in deren Mittenabschnitt einen radial nach außen vorragenden Vorsprung (in Fig. 4 nicht sichtbar) der axial mit einem radial nach innen vorragenden Vorsprung 33 in Anlage bringbar ist, um ein durch die Federeinrichtung 5 verursachtes Herausdrücken der Innenhülse 29 aus der Außenhülse 3 in Richtung proximal zu verhindern.

Die Innenhülse 29 dient daher quasi als axiales Distanzstück des werkzeugschaftseitigen Federsitzes A in die Zentriereinheit 1 hinein, wobei in diesem Fall die als Gleitlager fungierenden Innendurchmesser der Außenhülse 3 nichtmehr individuell an den Werzeugschaft W angepasst werden müssen, sondern für den Außendurchmesser der Innenhülse 29 als Bestandteil der Zentriereinheit 1 eingerichtet sind.

Abschließend sei darauf hingewiesen, dass alle vorstehend mit einer Zahl gekennzeichneten Bauteile Bestandteil der Zentriereinheit 1 sind, wohingegen alle mit einem Buchstaben gekennzeichneten Bauteile nicht Bestandteil der Zentriereinheit sind.

Die vorliegende Offenbarung betrifft zusammengefasst eine, zu medizinischen Zwecken vorgesehene und eingerichtete Zentriereinheit (1) für ein Einschraubwerkzeug (W) zum Einschrauben einer Madenschraube (M) in die Tulpe (T) einer Pedikelschraube (P), wobei die Zentriereinheit (1) eine Außenhülse (3) und eine in Axialrichtung der Außenhülse (3) wirkende Federeinrichtung (5) hat, die in die Außenhülse (3) eingelegt oder eingesetzt ist und die sich an einem als Federsitz dienenden Absatz an der Innenseite der Außenhülse (3) in Richtung distal abstützt.

## Patentansprüche

1. Zentriereinheit (1) für ein Montagewerkzeug für das Setzen einer Madenschraube in eine Tulpe einer Pedikelschraube mit einer Außenhülse (3) und einer in Axialrichtung der Außenhülse (3) wirkenden Federeinrichtung (5), die in die Außenhülse (3) eingelegt oder eingesetzt ist und die sich an einem Außenhülsen-innseitigen Absatz in Richtung distal abstützt, die Federeinrichtung (5) dafür vorgesehen und ausgebildet, eine axiale Vorspannkraft auf das in die Außenhülse (3) aus proximal eingeführtes Montagewerkzeug zum Aufsetzen und Festdrehen der Madenschraube auf oder in der Tulpe der Pedikelschraube auszuüben, sobald das Montagewerkzeug in der Außenhülse (3) über ein vorbestimmtes Maß axial verschoben wird, **dadurch gekennzeichnet, dass** die Außenhülse (3) eine distale Stirnkante hat, die dafür vorgesehen und ausgebildet ist, gegen die Tulpe axial angesetzt zu werden, derart, dass bei einer axialen Kraftbeaufschlagung des Montagewerkzeugs in Richtung distal das Montagewerkzeug gegen die Federkraft der Federeinrichtung (5) relativ zur Außenhülse (3) verschiebbar ist, soweit, dass die distale Montagewerkzeugspitze über die distale Stirnkante der Außenhülse (3) vorragt.

2. Zentriereinheit (1) nach Anspruch 1, mit einer Innenhülse (29), die am proximalen Endabschnitt der Außenhülse (3) axialverschiebbar eingesetzt oder einsetzbar ist und die sich in distaler Richtung gegen die Federeinrichtung (5) abstützt, wobei sich die Innenhülse (29) proximal an die Federeinrichtung (5) unmittelbar anschließt oder zumindest abschnittsweise in die Federeinrichtung (5) vorzugsweise in Form einer Schraubendruckfeder radial innseitig hineinragt, um so bevorzugt die Federeinrichtung (5) radial zu stützen.

3. Zentriereinheit (1) nach Anspruch 2, wobei die Innenhülse (29) mit wenigstens einem radial nach außen abstehenden Umfangsvorsprung ausgebildet ist, der als proximaler Axialanschlag für die Federeinrichtung (5) dient.

4. Zentriereinheit (1) nach Anspruch 2 oder 3, wobei die Innenhülse (29) mit einem radial nach außen vorragenden proximalen Vorsprung ausgebildet ist, der als Bewegungsanschlag in Richtung proximal dient und mit einer Hinterschneidung (33) an der radialen Innenseite der Außenhülse (3) in Anlage bringbar ist.

5. Zentriereinheit (1) nach einem der vorstehenden Ansprüche mit an der Außenhülse (3) an wenigstens zwei diametral sich gegenüberliegenden Umfangsabschnitten schwenkbar gelagerten Eingriffsarmen (17), die sich jeweils in Außenhülsen-Längsrichtung erstrecken und in ihren longitudinalen Mittenabschnitten wippenartig an einem jeweiligen Lagerbock (15) anscharniert sind, die an der radialen Außenseite der Außenhülse (3) fixiert oder ausgebildet sind, wobei jeder Eingriffsarm (15) mittels einer Feder (19) so vorgespannt ist, dass dessen distales Ende radial nach innen gedrückt wird.

6. Zentriereinheit (1) nach Anspruch 5, wobei jeder Eingriffsarm (17) einen distalen Eingriffsabschnitt (19) hat, der dafür vorgesehen und ausgebildet ist, mit der äußeren Umfangsseite der Tulpe form- und/oder reibschlüssig in Eingriff zu kommen.

7. Zentriereinheit (1) nach einem der vorstehenden, wobei die Innenseite der Außenhülse (3) in einem distalen Außenhülsenabschnitt einen radial aufgeweiteten Aufnahmeraum (21) zur Aufnahme der Madenhülse ausbildet, der sich vorzugsweise in Richtung proximal zu einem Außenhülsen-Axialabschnitt mit verengtem Innenradius verengt, der vorzugsweise dafür vorgesehen und ausgebildet ist, ein Gleitlager für den Werkzeugschaft oder die Innenhülse zu bilden.

8. Zentriereinheit (1) nach Anspruch 7, wobei der Außenhülsen-Axialabschnitt mit verengtem Innenradius an seinem proximalen Ende eine radiale Aufweitung ausbildet, wodurch sich eine nach proximal gerichtete Anlagekante (25) für die Federeinrichtung (5) ergibt.

9. Zentriereinheit (1) nach einem der vorstehenden Ansprüche, wobei ein proximaler Federanschlag insbesondere an der Innenhülse (29) so dimensioniert ist, dass er umfangsseitig gleitend an der Innenseite der Außenhülse (3) gleitend anliegt.

## Claims

1. A centering unit (1) for an assembly tool for setting a grub screw into a tulip of a pedicle screw, having an outer sleeve (3) and a spring device (5) which acts in the axial direction of the outer sleeve (3) and which is inserted or placed into the outer sleeve (3) and which is supported on a shoulder on the inner surface of the outer sleeve in the distal direction, wherein the spring device (5) is provided and configured to exert an axial pre-tensioning force on the assembly tool inserted into the outer sleeve (3) from the proximal direction for setting and tightening the grub screw on or in the tulip of the pedicle screw as soon as the assembly tool is axially displaced in the outer sleeve (3) over a predetermined amount,
**characterized in that**
the outer sleeve (3) has a distal end edge which is provided and configured to be axially set against the tulip in such a way that, when an force is axially applied to the assembly tool in the distal direction, the assembly tool is displaceable relative to the outer sleeve (3) against the spring force of the spring device (5) to such an extent that the distal tip of the assembly tool projects beyond the distal end edge of the outer sleeve (3).

2. The centering unit (1) according to Claim 1, having an inner sleeve (29) which is inserted or insertable in an axially displaceable manner at the proximal end portion of the outer sleeve (3) and which is supported in the distal direction against the spring device (5), wherein the inner sleeve (29) directly adjoins the spring device (5) in the proximal direction or projects radially on the inside at least in portions into the spring device (5), preferably in the form of a helical compression spring, in order to thus preferably support the spring device (5) radially.

3. The centering unit (1) according to Claim 2, wherein the inner sleeve (29) is configured with at least one circumferential projection which projects radially outward and which serves as a proximal axial stop for the spring device (5).

4. The centering unit (1) according to Claim 2 or 3, wherein the inner sleeve (29) is configured with a proximal projection which projects radially outward and which serves as a movement stop in the proximal direction and can be brought into contact with an undercut (33) on the radial inner side of the outer sleeve (3).

5. The centering unit (1) according to one of the preceding claims, with engagement arms (17) which are pivotably mounted on the outer sleeve (3) on at least two diametrically opposite circumferential portions and which each extend in the longitudinal direction of the outer sleeve and are hinged in the manner of a rocker in their longitudinal central portions on a respective bearing block (15) and which are fixed or configured on the radial outer side of the outer sleeve (3), wherein each engagement arm (15) is prestressed via a spring (19) such that its distal end is pressed radially inward.

6. The centering unit (1) according to Claim 5, wherein each engagement arm (17) has a distal engagement portion (19) which is provided and configured to come into form-fitting and/or frictional engagement with the outer circumferential side of the tulip.

7. The centering unit (1) according to one of the preceding claims, wherein the inner side of the outer sleeve (3) forms, in a distal outer sleeve portion, a radially widened receiving space (21) for receiving the grub sleeve, wherein the receiving space preferably narrows in the proximal direction with respect to an outer sleeve axial portion with a narrowed inner radius which is preferably provided and configured to form a plain bearing for the tool shank or the inner sleeve.

8. The centering unit (1) according to Claim 7, wherein the outer sleeve axial portion with a narrowed inner radius forms a radial widening at its proximal end, as a result of which a proximally directed contact edge (25) for the spring device (5) is created.

9. The centering unit (1) according to one of the preceding claims, wherein a proximal spring stop, in particular on the inner sleeve (29), is dimensioned such that it bears in a sliding manner on the inner side of the outer sleeve (3) on the circumferential side.

## Revendications

1. Unité de centrage (1) pour un outil de montage pour la mise en place d'une vis sans tête dans une tulipe d'une vis pédiculaire avec une gaine extérieure (3) et un dispositif à ressort (5) agissant dans la direction axiale de la gaine extérieure (3), dispositif qui est placé ou inséré dans la gaine extérieure (3) et qui s'appuie dans la direction distale sur un épaulement à l'intérieur de la gaine extérieure, le dispositif à ressort (5) étant prévu et conçu pour exercer une force de précontrainte axiale sur l'outil de montage introduit de manière proximale dans la gaine extérieure (3) pour la mise en place et le vissage de la vis sans tête sur ou dans la tulipe de la vis pédiculaire dès que l'outil de montage est déplacé axialement dans la gaine extérieure (3) d'une distance prédéterminée,
**caractérisé en ce que**
la gaine extérieure (3) présente une arête avant distale qui est prévue et conçue pour être placée axialement contre la tulipe, de telle sorte que, lors d'une application de force axiale de l'outil de montage dans la direction distale, l'outil de montage est déplaçable à l'encontre de la force de ressort du dispositif à ressort (5) par rapport à la gaine extérieure (3), de manière à ce que la pointe d'outil de montage distale dépasse de l'arête avant distale de la gaine extérieure (3).

2. Unité de centrage (1) selon la revendication 1, avec une gaine intérieure (29) qui est insérée ou peut être insérée de manière à pouvoir se déplacer axialement au niveau de la partie d'extrémité proximale de la gaine extérieure (3) et qui s'appuie dans la direction distale contre le dispositif à ressort (5), dans laquelle la gaine intérieure (29) se raccorde directement de manière proximale au dispositif à ressort (5) ou fait saillie radialement à l'intérieur au moins par sections dans le dispositif à ressort (5) de préférence sous la forme d'un ressort de compression hélicoïdal pour ainsi soutenir radialement de préférence le dispositif à ressort (5).

3. Unité de centrage (1) selon la revendication 2, dans laquelle la gaine intérieure (29) est conçue avec au moins une saillie périphérique s'étendant radialement vers l'extérieur, qui sert de butée axiale proximale pour le dispositif à ressort (5).

4. Unité de centrage (1) selon la revendication 2 ou 3, dans laquelle la gaine intérieure (29) est conçue avec une saillie proximale faisant saillie radialement vers l'extérieur, qui sert de butée de course dans la direction proximale et qui peut venir en appui avec une contre-dépouille (33) au niveau de la face intérieure radiale de la gaine extérieure (3).

5. Unité de centrage (1) selon l'une quelconque des revendications précédentes avec des bras de préhension (17) logés de manière pivotante au niveau de la gaine extérieure (3) au niveau d'au moins deux sections périphériques diamétralement opposées, qui s'étendent respectivement dans la direction longitudinale de la gaine extérieure et sont articulés dans leurs sections médianes longitudinales à la manière d'une balançoire au niveau d'un support de palier respectif (15), qui sont fixés ou conçus au niveau de la face extérieure radiale de la gaine extérieure (3), dans laquelle chaque bras de préhension (15) est précontraint au moyen d'un ressort (19) de sorte que l'extrémité distale de celui-ci soit poussée radialement vers l'intérieur.

6. Unité de centrage (1) selon la revendication 5, dans laquelle chaque bras de préhension (17) présente une section de préhension distale (19) qui est prévue et conçue pour se mettre en prise par complémentarité de formes et/ou par friction avec la surface périphérique extérieure de la tulipe.

7. Unité de centrage (1) selon l'une quelconque des revendications précédentes, dans laquelle la face intérieure de la gaine extérieure (3) conçoit dans une section de gaine extérieure distale un espace de logement (21) élargi radialement destiné au logement de la gaine sans tête qui se rétrécit de préférence dans la direction proximale vers une section axiale de gaine extérieure avec un rayon intérieur rétréci, qui est de préférence prévue et conçue pour former un palier lisse pour la tige d'outil ou la gaine intérieure.

8. Unité de centrage (1) selon la revendication 7, dans laquelle la section axiale de gaine extérieure conçoit avec un rayon intérieur rétréci au niveau de son extrémité proximale un élargissement radial, ce qui donne lieu à une arête d'appui (25) orientée de manière proximale pour le dispositif à ressort (5).

9. Unité de centrage (1) selon l'une quelconque des revendications précédentes, dans laquelle une butée de ressort proximale en particulier au niveau de la gaine intérieure (29) est dimensionnée de sorte qu'elle repose, de manière coulissante sur sa périphérie, au niveau de la face intérieure de la gaine extérieure (3).
